(19) Europäisches Patentamt
European Patent Office
Office européen des brevets

(11) **EP 3 428 622 B1**

(12) **EUROPÄISCHE PATENTSCHRIFT**

(45) Veröffentlichungstag und Bekanntmachung des Hinweises auf die Patenterteilung:
**24.02.2021 Patentblatt 2021/08**

(21) Anmeldenummer: **18168719.5**

(22) Anmeldetag: **23.04.2018**

(51) Int Cl.:
*G01N 21/77* *(2006.01)*    *G01N 33/543* *(2006.01)*
*G02B 6/34* *(2006.01)*

(54) **DIFFRAKTIVER BIOSENSOR**

DIFFRACTIVE BIOSENSOR

BIOCAPTEUR DIFFRACTIF

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **12.07.2017 DE 102017211910**

(43) Veröffentlichungstag der Anmeldung:
**16.01.2019 Patentblatt 2019/03**

(73) Patentinhaber: **Dr. Johannes Heidenhain GmbH 83301 Traunreut (DE)**

(72) Erfinder:
• **HOLZAPFEL, Wolfgang**
  **83119 Obing (DE)**
• **KUGLER, Michael**
  **83368 St. Georgen (DE)**
• **SCHADE, Marco**
  **83278 Traunstein (DE)**

(56) Entgegenhaltungen:
**EP-B1- 0 226 604         WO-A1-97/37211
WO-A1-2010/063116    US-A1- 2014 080 729
US-B1- 7 505 641**

EP 3 428 622 B1

**Beschreibung**

**GEBIET DER TECHNIK**

Die vorliegende Erfindung betrifft einen diffraktiven Biosensor. Solche Sensoren basieren auf der Adsorption zu detektierender Biomoleküle auf einem Wellenleiter, wobei die Biomoleküle ein diffraktives Gitter zum Ein- oder Auskoppeln von Licht in den Wellenleiter bilden. Das Signal eines Lichtdetektors dient als Maß für die Massenbelegung des Biosensors mit den Biomolekülen.

**STAND DER TECHNIK**

Aus der Optik sind auf einem Substrat angeordnete Wellenleiter bekannt, die ein optisches Gitter zum Ein- oder Auskoppeln von Licht aufweisen. Bei so einem optischen Gitter handelt es sich zum Beispiel um in das Substrat oder in den Wellenleiter geätzte Strukturen, die somit aus dem Material des Substrats bzw. des Wellenleiters bestehen. Die benötigte Gitterperiode hängt von der Wellenlänge des verwendeten Lichtes und vom Brechungsindex des Wellenleiters ab. Die Gitterperiode liegt abhängig vom Kopplungswinkel im Bereich der effektiven Wellenlänge des Lichtes im Wellenleiter. Typischerweise beträgt sie etwa die halbe Vakuumwellenlänge des Lichtes. Derart feine Strukturen bedeuten einen großen Herstellungsaufwand.

Im Bereich der Biosensoren sind auch Gitter zum Ein- und Auskoppeln von Licht bekannt, die aus biologischer Materie bestehen und als Rezeptor für die zu untersuchenden Biomoleküle wirken. Lagern sich solche Biomoleküle an den zum Gitter strukturierten Rezeptoren an, formen die Biomoleküle ein optisch wirksames Gitter. Solche zum Gitter strukturierten Rezeptoren mit oder ohne adsorbierten Biomolekülen werden im Folgenden als Biogitter oder einfacher als Gitter bezeichnet. Da die Beugungseffizienz so eines Gitters von der Massenbelegung des Gitters mit den Biomolekülen abhängt, kann anhand der mittels eines Detektors gemessenen Intensität des gebeugten Lichtes eine quantitative Aussage über die Massenbelegung getroffen werden.

Aus der US 20140080729 A1 ist ein diffraktiver Biosensor zum selektiven Nachweis von Biomolekülen bekannt, bei dem kollimiertes Licht auf ein in ein Substrat geätztes Einkoppelgitter in einem flächigen Wellenleiter trifft. Die Auskopplung des Lichts erfolgt an einer Kante des Wellenleiters.

Aus der WO 2010063116 A1 ist ein diffraktiver Biosensor bekannt. Die Schrift lehrt, dass anstelle eines geätzten Gitters eine periodische Anordnung von Rezeptoren für Biomoleküle verwendet werden kann.

Die US 7505641 B1 und die WO 9737211 A1 offenbaren weitere Biosensoren, die auf der Wechselwirkung von Licht und optischen Gittern beruhen, wobei die Wechselwirkung von der Konzentration der untersuchten Biomoleküle abhängt.

Aus der WO 2015004264 A1 ist ein diffraktiver Biosensor bekannt, bei dem divergentes Licht durch ein Substrat auf ein optisches Gitter zum Einkoppeln von Licht in einen Wellenleiter fällt. Das im Wellenleiter propagierende Licht trifft dann auf ein Gitter, das als Auskoppelgitter wirkt. Das ausgekoppelte Licht wird durch das Substrat hindurch auf einen Detektor fokussiert. Die im Detektor gemessene Lichtintensität ist ein Maß für die Belegung des Auskoppelgitters mit dem zu untersuchenden Biomolekül. Die Verwendung von zwei Biogittern bedeutet allerdings ein sehr geringes Signal durch die zweifache schwache Kopplung.

Aus der WO 2013107811 A1 ist ein diffraktiver Biosensor bekannt, bei dem ein Biogitter und ein in den Wellenleiter geätztes Einkoppelgitter benutzt wird Dies bedeutet wegen dem zusätzlichen Lithographieschritt aber einen erheblichen Mehraufwand bei der Herstellung solcher Biosensoren.

Aus der EP 0226604 B1 ist aus der Figur 2 ein diffraktiver Biosensor bekannt, bei dem durch ein Substrat einfallendes Licht über ein optisches Gitter in einen Wellenleiter eingekoppelt wird. Im Wellenleiter propagiert das einfallende Licht direkt zu einem Detektor, der an einer Kante des Wellenleiters angeordnet ist. Ein separates Auskoppelgitter wird hier nicht benötigt. Bei diesem Biosensor beruht die quantitative Analyse der zu untersuchenden Biomoleküle auf eine Änderung der Brechzahl des Mediums oberhalb des optischen Gitters. Diese Brechzahländerung bewirkt eine der Konzentration der Biomoleküle proportionale Änderung der Lichtintensität im Detektor. Solche optischen Gitter, deren Beugungseffizienz durch eine lediglich in der Nähe des Gitters auftretende Brechzahländerung beeinflusst wird, sind aber weniger gut geeignet als die oben erwähnten Gitter, bei denen das beugende Gitter durch Anlagerung von zu untersuchenden Biomolekülen entsteht. Grund hierfür ist, dass das Nullsignal groß ist und die flächige Aufbringung der Biomoleküle auf Stege und Lücken oberhalb des optischen Gitters zu einer geringen Empfindlichkeit führt.

**ZUSAMMENFASSUNG DER ERFINDUNG**

Es ist daher Aufgabe der Erfindung, ein optimiertes Design eines diffraktiven Biosensors anzugeben, der kostengünstig hergestellt werden kann, und dennoch eine gute Detektion von zu untersuchenden Biomolekülen erlaubt.

Diese Aufgabe wird gelöst durch eine Vorrichtung gemäß Anspruch 1. Vorteilhafte Details dieser Vorrichtung ergeben sich auch aus den von Anspruch 1 abhängigen Ansprüchen.

Es wird ein diffraktiver Biosensor zum selektiven Nachweis von Biomolekülen offenbart, mit einem Substrat und einem auf dem Substrat angeordneten flächigen Wellenleiter. Einfallendes Licht wird mittels eines optischen Gitters in den Wellenleiter eingekoppelt und zu einem hinter einer Kante des Wellenleiters angeordneten Detektionsbereich geleitet, wobei die Effizienz der Einkopplung und damit die Intensität des im Detektionsbereich eintreffenden Lichtes von einer Massenbelegung des Gitters mit den nachzuweisenden Biomolekülen abhängt. Das Gitter weist auf dem Wellenleiter periodisch angeordnete Rezeptoren für die Biomoleküle auf, und das auf das Gitter einfallende Licht ist kollimiert.

Eine nicht erfindungsgemäße Möglichkeit besteht darin, dass das optische Gitter ein lineares Gitter ist und kollimiertes Licht zum Detektionsbereich in den Wellenleiter sendet, und dass eine erste Linse zwischen der Kante des Wellenleiters und dem Detektionsbereich das Licht auf den Detektionsbereich fokussiert. Erfindungsgemäß weist das optische Gitter gekrümmte Gitterlinien auf und fokussiert das Licht in Richtung des Detektionsbereiches.

Die vorliegende Erfindung sieht also ein diffraktives Biogitter mit gekrümmten Linien vor, welches kollimiert einfallendes Anregungslicht bioselektiv in einen planaren Wellenleiter einkoppelt und gleichzeitig auf einen Detektionsbereich an oder in der Nähe der Kante des Wellenleiters fokussiert.

Das mit einem Lichtdetektor gemessene Signal dient als Maß für die Massenbelegung der adsorbierten Biomoleküle. Hierzu wird auf ein geeignetes Substrat ein planarer Wellenleiter aufgebracht, dessen Brechungsindex größer sein muss als der des Substrats. Auf der Oberfläche des Wellenleiters bildet sich durch Adsorption von Biomolekülen entlang definierter Linien ein diffraktives Biogitter. Zur Herstellung des Biosensors müssen also geeignete Rezeptoren für die nachzuweisenden Biomoleküle zum Gitter strukturiert auf den Wellenleiter aufgebracht werden.

Für die Gitter mit gekrümmten Linien gilt, dass die von allen Gitterorten ausgehenden Teilstrahlen im Fokus (also im Detektionsbereich) konstruktiv interferieren sollen. Daraus ergibt sich für die $x_j$, $y_j$-Koordinaten dieser Gitterlinien folgende Formel:

$$x_j(y_j) = \frac{f N_{\text{eff}}^2 + n\lambda\sin(\alpha)(j + j_0) + N_{\text{eff}}\sqrt{(\lambda(j + j_0) + nf\sin(\alpha))^2 - y_j^2\left(N_{\text{eff}}^2 - n^2\sin^2(\alpha)\right)}}{N_{\text{eff}}^2 - n^2\sin^2(\alpha)}$$

Für senkrechten Einfall ($\alpha=0°$) vereinfacht sich diese Formel wie folgt:

$$x_j(y_j) = f + \sqrt{\frac{\lambda^2}{N_{\text{eff}}^2}(j + j_0)^2 - y_j^2}$$

Hierbei bezeichnet Neff den effektiven Brechungsindex der geführten Mode im Wellenleiter, n den Brechungsindex des Mediums aus dem der Lichtstrahl einfällt, $\lambda$ die Wellenlänge des Lichts im Vakuum, f die Brennweite des Gitters in der x-y-Ebene, j einen ganzzahligen Zählindex, $j_0$ einen beliebig gewählten ganzzahligen Offset und $\alpha$ den Winkel des einfallenden Lichts gemessen zur Flächennormalen des Wellenleiters, wobei der Einfallswinkel positiv gezählt wird in positive x-Richtung, die vom Detektionsbereich in Richtung des Gitters verläuft.

Im allgemeinen Fall besteht die Gitterstruktur aus einer Schar von Ellipsen, der normale Abstand $\Lambda$ benachbarter Linien ist annähernd äquidistant: bei y=0 beträgt der Abstand $\Lambda = \lambda/(N_{\text{eff}} - n*\sin(\alpha))$, für y#0 verringert sich der normale Abstand geringfügig aufgrund der gekrümmten Linienstruktur. Im speziellen Fall $\alpha=0°$ vereinfacht sich die Gitterstruktur zu einer Schar aus konzentrischen Kreisen um einen Punkt im Abstand f, und der normale Abstand benachbarter Linien ist mit $\lambda = \lambda/N_{\text{eff}}$ für alle y streng äquidistant. Diese äquidistante Anordnung der Linien ist besonders vorteilhaft, da sie den lithographischen Herstellungsprozess deutlich vereinfacht. Die Herstellung des Biogitters kann lithographisch durch Strukturierung einer Schicht geeigneter Rezeptoren erfolgen.

Das Biogitter wird mit kohärentem, kollimiertem Anregungslicht (z.B. das Licht eines Lasers) beleuchtet. Die Verwendung von kollimiertem Anregungslicht hat den Vorteil, dass nur der Winkel zwischen Biogitter und einfallendem Lichtstrahl korrekt ausgerichtet werden muss. Dies ist bedeutend einfacher als eine in allen drei Raumrichtungen genaue Positionierung einer divergenten Lichtquelle - z.B. eines Faserendes - im Brennpunkt eines passend ausgestalteten Biogitters zur Einkopplung von divergentem Licht. Außerdem erfordert die Verwendung von divergent einfallendem Licht aufgrund des Winkelspektrums stets einen kontinuierlich variierenden Linienabstand der Gitterlinien, während für kollimiertes Licht ein äquidistantes Gitter ausreichend ist.

In Gegenwart von adsorbierten Biomolekülen an der Rezeptor-Oberfläche kommt es zu einer bioselektiven Einkopplung des Anregungslichts in den Wellenleiter, wo das eingekoppelte Signallicht durch Totalreflektion geführt wird. Durch die Krümmung der Gitterlinien wird das Signallicht auf einen Punkt im Detektionsbereich an oder in der Nähe der Kante des Wellenleiters fokussiert. Eine Einschnürung des Lichts senkrecht zur Ebene des Wellenleiters ist intrinsisch durch den Wellenleiter selbst gegeben.

Eine zur Auskopplung des Lichts geeignete Kante des Wellenleiters mit hinreichend geringer Rauheit ist z.B. durch Laserschneiden oder Sägen mit anschließender Politur realisierbar.

Weitere Vorteile und Einzelheiten der vorliegenden Erfindung ergeben sich aus der nachfolgenden Beschreibung verschiedener Ausführungsformen anhand der Figuren.

## KURZE BESCHREIBUNG DER ZEICHNUNGEN

Dabei zeigt

Figur 1        eine erste Ausführungsform mit schrägem Lichteinfall,

Figur 2        eine zweite Ausführungsform mit senkrechtem Lichteinfall,

Figur 3        eine dritte Ausführungsform mit einer regelmäßigen Gittermatrix und zeitlichem Multiplexing,

Figur 4a       eine vierte Ausführungsform mit versetzt angeordneten Gittern in einer Matrix,

Figur 4b       eine Alternative zur vierten Ausführungsform mit einer regelmäßigen Gittermatrix mit versetzten Brennpunkten,

Figur 5        ein nicht erfindungsgemäßes Beispiel mit einer Matrix von Gittern mit geraden Gitterstrichen,

Figur 6        eine fünfte Ausführungsform mir einem Detektor im Detektionsbereich,

Figur 7        eine sechste Ausführungsform mit einer Zylinderlinse vor dem Detektor,

Figur 8        eine siebte Ausführungsform mit einer sphärischen oder asphärischen Linse vor dem Detektor,

Figur 9        eine achte Ausführungsform mit einer Lichtleitfaser,

Figur 10       eine neunte Ausführungsform mit Blenden zur Filterung von Streulicht,

Figur 11       eine zehnte Ausführungsform mit einer Blende in einer Fourier-Zwischenebene,

Figur 12       eine elfte Ausführungsform mit einer Spiegelschicht zur Erhöhung der Einkoppeleffizienz.

## BESCHREIBUNG DER AUSFÜHRUNGSFORMEN

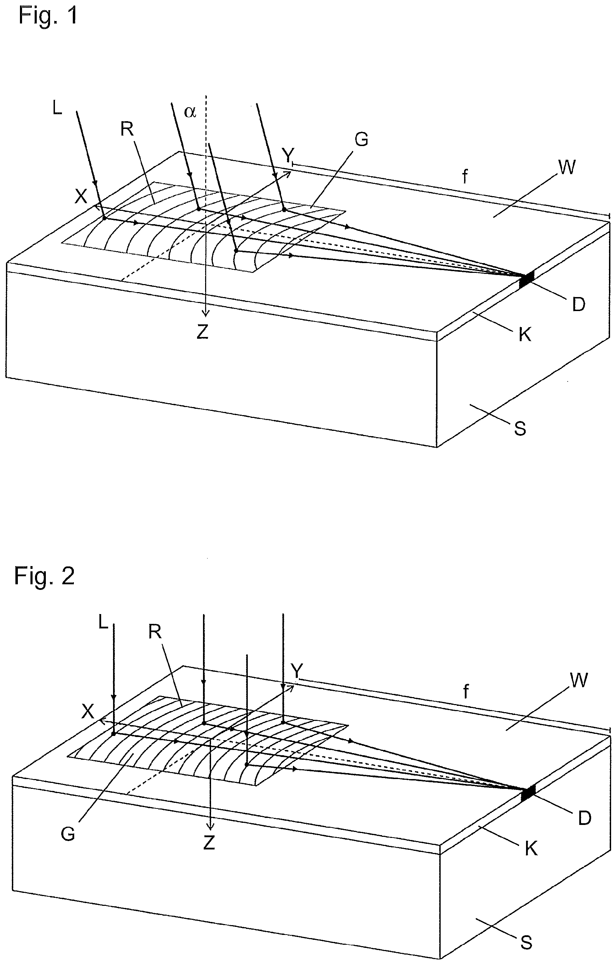

Die Figur 1 zeigt eine erste Ausführungsform eines diffraktiven Biosensors. Auf einem Substrat S ist in der X-Y-Ebene ein flächiger Wellenleiter W angeordnet. Der Brechungsindex des Wellenleiters W muss größer sein als der Brechungsindex des Substrats S. Geeignete Materialien für das Substrat S wären Glas oder ein Polymer wie z.B. Polyethylen mit einem Brechungsindex von ungefähr 1,5. Als Wellenleiter W kann $Ta_2O_5$, $Si_3N_4$, $SiO_xN_y$, $TiO_2$ oder SiC dienen, jeweils mit einem Brechungsindex von 2,12, 2,04, 1,5 bis 2,1, 2,58 bzw. 2,63.

Auf dem Wellenleiter W ist ein Gitter G aus linienförmig strukturierten Rezeptoren R angeordnet. Die Rezeptoren R sind geeignet, die nachzuweisenden Biomoleküle zu adsorbieren und dadurch ein optisch wirksames Gitter G zum Einkoppeln von Licht L in den Wellenleiter W zu bilden. Das Gitter G ist also ein Biogitter im Sinne der oben gegebenen Definition.

Das Gitter G fokussiert das kollimiert einfallende Licht L auf einen Detektionsbereich D, der an oder in der Nähe einer Kante K des Wellenleiters W liegt. Hier kann wie später noch gezeigt wird, das Licht L detektiert oder zu einem Detektor weitergeleitet werden. Die Brennweite f des Gitters G ist so gewählt, dass sie dem Abstand vom Gitter G zum Detektionsbereich D entspricht.

In dieser ersten Ausführungsform ist der Einfallswinkel $\alpha$ des Lichts L größer 0° (flacher Einfall) gewählt. Dies hat den Vorteil, dass der Abstand zwischen benachbarten Linien von Rezeptoren R größer ist als im Fall $\alpha$=0°, was den lithographischen Herstellungsprozess vereinfacht. Des Weiteren ist die Bedingung für die Einkopplung des Lichtes L in den Wellenleiter W nur in Vorwärtsrichtung erfüllt, sodass die Einkopplung in Rückwärtsrichtung im Gegensatz zum Fall $\alpha$=0° unterdrückt wird.

Eine im Vergleich zur Figur 1 abgewandelte Ausführungsform wäre ein Einfallswinkel kleiner als 0° (spitzer Einfall), also mit einfallenden Lichtstrahlen L, deren Richtungskomponente in der X-Richtung gegenüber der Figur 1 umgekehrt ist. Diese Änderung bedingt zwar eine Verringerung des Abstands $\Lambda=\lambda/(N_{eff}- n*\sin(\alpha))$ zwischen benachbarten Linien, hat aber den Vorteil, dass einfallendes Licht nicht in Richtung des an der Kante K befindlichen Detektionsbereichs D gestreut werden kann.

Die Figur 2 zeigt ein weiteres Ausführungsbeispiel, bei dem der Einfallswinkel nun $\alpha=0°$ (senkrechter Einfall) gewählt wurde. Falls eine zusätzliche Einkopplung des Lichtes L in die Rückwärtsrichtung toleriert werden kann, ist diese Ausführung besonders vorteilhaft, da sich eine Gitterstruktur mit streng äquidistanten Kreissegmenten um den Fokuspunkt des Gitters G ergibt. Dies vereinfacht die Lithographie, da für ein äquidistantes Gitter bei gegebener Belichtungswellenlänge eine vollständige Unterdrückung der nullten Ordnung der notwendigen Lithographie-Phasenmaske möglich wird, was den Kontrast zwischen Steg und Lücke maximiert.

Es folgen Ausführungsbeispiele, die mehrere Gitter G auf einem Biosensor verwenden, um mit einem einzigen Biosensor mehrere Proben oder eine oder mehrere Proben auf verschiedene Biomoleküle testen zu können. Dies kann bestimmte Analyse-Aufgaben erheblich beschleunigen.

Diese Ausführungsbeispiele sehen ein Multiplexing mit einem zweidimensionalen Array bestehend aus m Zeilen von Gittern nebeneinander parallel zur Auskopplungskante K und n Spalten senkrecht dazu vor, was dadurch möglich wird, dass die Gitter G nur schwach mit Licht L im Wellenleiter W wechselwirken. Dadurch kann Signallicht L, das bioselektiv an einem Gitter G eingekoppelt wurde, durch ein nachfolgendes Gitter G in derselben Spalte hindurchpropagieren, ohne dass hierdurch die Stärke des Signallichts nennenswert beeinflusst wird. Selbst eine Monolage aus Biomolekülen hätte (z.B. für TSH, einen typischen Vertreter der zu detektierenden Biomoleküle) nur eine Beugungseffizienz von etwa $10^{-4}$, die realistische Belegung von Biosensoren und damit die Beugungseffizienz liegen typischerweise noch um Größenordnungen niedriger.

Das Multiplexing kann unterschiedlich ausgestaltet werden, was dazu passende Detektionsvarianten an der Kante K, an der das Licht L ausgekoppelt wird, erfordert. Grundsätzlich ist die Trennung des signaltragenden Lichts L an der Kante K, das von unterschiedlichen Gittern G erzeugt wurde, auf drei unterschiedliche Arten möglich:

a) Zeitlich, d.h. durch sequentielles Beleuchten der einzelnen Gitter G.

b) Im Ortsraum, indem man den Fokuspunkt der einzelnen Gitter G an unterschiedliche Orte auf der Wellenleiterkante K setzt.

c) Der Richtung nach, indem man den Raumwinkel, in den ein Gitter G emittiert so gestaltet, dass er sich von den anderen Gittern G unterscheidet;

und natürlich Kombinationen dieser drei Möglichkeiten.

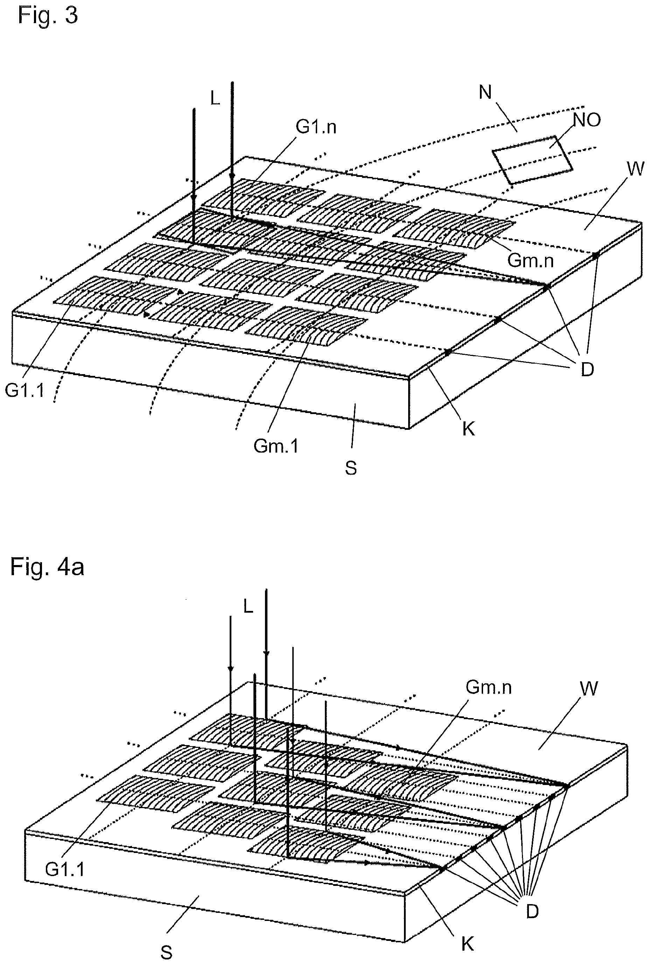

Eine in der Figur 3 gezeigte dritte Ausführungsform nach Variante a) besteht aus einer Anordnung von m x n Gittern Gm.n in m Zeilen und n Spalten in einem regelmäßigen Raster, wobei alle m Gitter Gm.n einer Spalte auf denselben Punkt an der Kante K fokussiert sind. Sequentielle Beleuchtung kann z.B. durch eine Blende N (Nipkow-Scheibe) mit passenden m Öffnungen NO von der Größe eines Gitters Gm.n erreicht werden, welche die Gitter Gm.n zeilenweise beleuchten, so dass ein Detektor an der Kante K das Licht L der verschiedenen Gitter Gm.n nacheinander empfängt. Eine Abschattung aller Gitter Gm.n bis auf jeweils eines zu jeder Zeit ist gleichermaßen durch ein transmittierendes LCD-Element möglich. Ein Vorteil des zeitlichen Multiplexings ist, dass kein Streulicht von anderen Gittern Gm.n oder unbelegten Stellen auf dem Wellenleiter W zum Signal des gerade beleuchteten Gitters Gm.n beiträgt, da keine anderen Stellen beleuchtet werden. Des Weiteren sinkt der Detektionsaufwand, da nur noch ein Detektor für eine schmale Stelle der Wellenleiterkante K vorgesehen werden muss, während andere Ausführungen ggf. eine vollständige Abbildung der gesamten Kante notwendig machen. In der Regel wird der Zeitaufwand für die Messung im Wesentlichen durch die auf Zeitskalen von Minuten stattfindende Reaktionsdynamik der Anlagerung der zu detektierenden Biomoleküle bestimmt. Die Integrationszeit zum Nachweis des eingekoppelten Signals fällt hier kaum ins Gewicht, daher kann der Zeitaufwand für sequentielle Messung unterschiedlicher Gitter Gm.n toleriert werden.

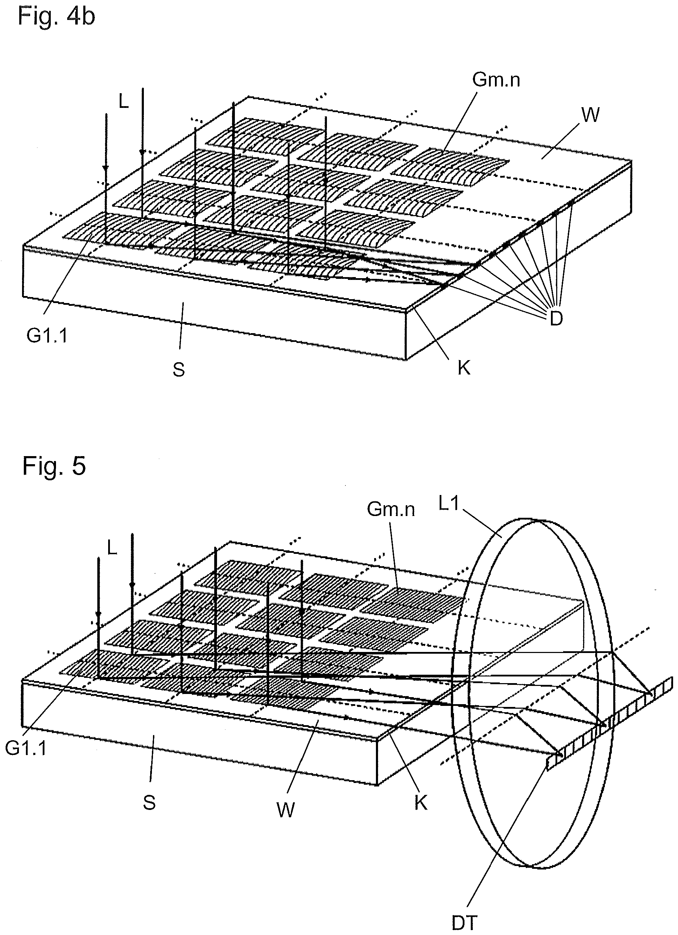

Die Figuren 4a und 4b zeigen Varianten einer vierten Ausführungsform, bei der nach Variante b) die Trennung des Lichtes L im Ortsraum vorgenommen wird. Gemäß Figur 4a sind die Gitter Gm.n in den einzelnen Zeilen leicht gegeneinander versetzt angeordnet, so dass das Licht L jeweils auf räumlich getrennte Detektionsbereiche D fällt. Als Detektor kann ein Zeilenarray von lichtempfindlichen Elementen dienen, das entlang der Kante K angeordnet wird. Vorteil dieser Ausführungsform ist, dass die Gitterstruktur spiegelsymmetrisch um die jeweilige optische Achse bleibt, was für die Optimierung der Lithographiemaske vorteilhaft ist.

Demgegenüber kann in der Variante nach Figur 4b durch ein verändertes Design der Gitter Gm.n jeweils ein anderer Fokuspunkt bzw. Detektionsbereich D anvisiert werden, der von allen anderen Fokuspunkten getrennt liegt. Vorteil dieser Ausführung ist, dass das regelmäßige Raster der Gitter Gm.n erhalten bleibt und alle Gitter Gm.n einer Spalte auf einer Linie senkrecht zur Substratkante K liegen. Dies vereinfacht z.B. die Lokalisierung der Gitter Gm.n bei den Herstellungsschritten, bei der Qualitätssicherung und bei der Probenaufbringung. Für den Versatz des Fokus der einzelnen Gitter Gm.n weg von der ursprünglichen optischen Achse x=0 ist eine triviale Modifikation der Formel zur Bestimmung der Gitterlinien (siehe oben) notwendig, die sich wie die Ursprungsformel aus der Forderung ergibt, dass die von allen Gitterorten ausgehenden Teilstrahlen im Fokus konstruktiv interferieren sollen.

Für die beiden Varianten des vierten Ausführungsbeispiels nach den Figuren 4a und 4b gilt, dass alle Gitter Gm.n einer Zeile aufgrund der Möglichkeit, die Brennweite und Einkopplungsrichtung identisch zu wählen, eine identische Struktur aufweisen können und so mit derselben Maske, auf der die jeweilige Struktur nur einmal vorhanden sein muss, nacheinander strukturiert werden können.

Die Figur 5 zeigt als nicht erfindungsgemäßes Beispiel die dritte Variante c) gemäß obiger Aufzählung von Möglichkeiten zur Trennung des Lichts L an der Kante K:

Eine Ausführungsform nach Variante c) ist eine m x n Anordnung von Gittern Gm.n, bei dem die Gitter das eingekoppelte Licht in jeweils unterschiedliche Richtungen in den Wellenleiter W koppeln, dabei aber nicht im Ortsraum fokussieren. Dieser Spezialfall, bei dem die Brennweite der Gitter Gm.n gegen unendlich geht, resultiert in besonders einfach zu fertigenden linearen Gittern Gm.n. Die Gitterstriche der Gitter Gm.n sind gemäß diesem Ausführungsbeispiel in unterschiedliche Richtungen gedreht. Von jedem der Gitter Gm.n läuft ein paralleles Lichtbündel in Richtung der Kante K. Durch Fokussierung der in unterschiedliche Richtungen propagierenden Lichtbündel mittels einer hinter der Kante K angeordneten Linse L1 und Positionierung eines Zeilendetektors DT in deren Brennebene (Fourierebene) kann das Licht L der unterschiedlichen Gitter Gm.n getrennt detektiert werden.

Es lässt sich zeigen, dass Varianten b) und c) vom Signal-Rausch Verhältnis grundsätzlich äquivalent sind, da ihr Modenvolumen ("Platzbedarf") im in diesem Fall zweidimensionalen Orts-Richtungs-Phasenraum aufgrund der Etendue-Erhaltung immer gleich ist. Ein Vorteil solcher Anordnungen ist, dass im Gegensatz zum zeitlichen Multiplexing die Nipkow-Scheibe bzw. die Vorrichtung zur gezielten Beleuchtung einzelner Gitter per LCD-Abschattung oder eine ähnliche Einrichtung eingespart wird.

Bei der Detektion des eingekoppelten Signallichts L an der Kante K des Wellenleiters können die Abbildung, die Sensorgeometrie und die Filterung des Lichts unterschiedlich ausgestaltet werden. Im Folgenden werden einige Beispiele hierfür aufgezeigt.

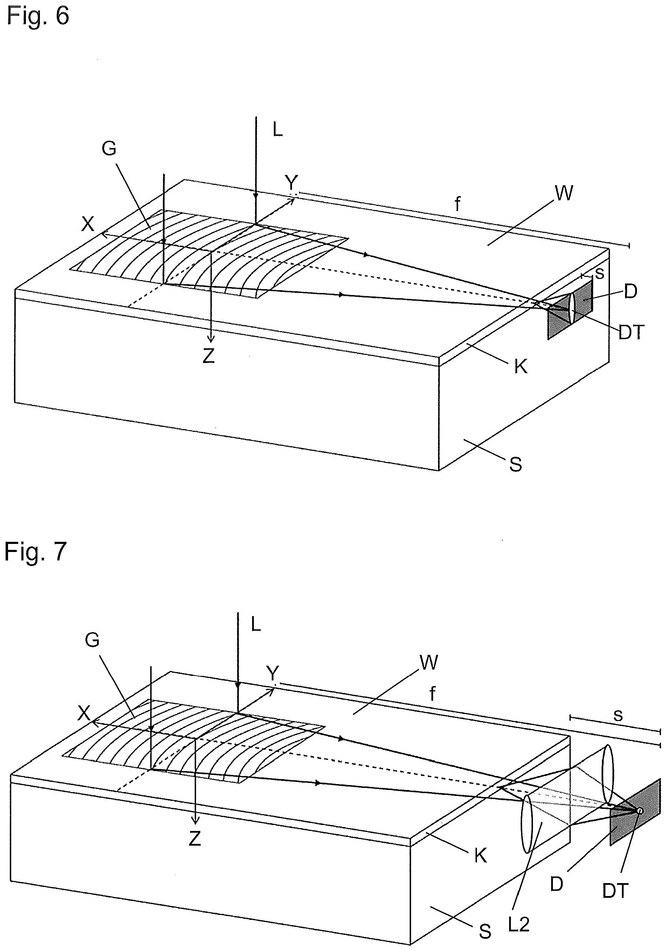

Figur 6 zeigt eine linsenlose Abbildung auf den Detektionsbereich D. In dieser Variante wird die Brennweite f des Gitters G so gewählt, dass das austretende Licht L nicht direkt auf die Kante, sondern etwas dahinter auf einen lichtempfindlichen Detektor DT fokussiert wird, welcher sich in möglichst geringem Abstand s (z.B. zwischen 10 und 100 Mikrometer) zur Kante K des Wellenleiters W befindet. Der Vorteil dieser Variante liegt im einfachen, robusten und kostengünstigen Aufbau, da keinerlei zusätzliche optische Komponenten benötigt werden. Da die numerische Apertur dieser Abbildung annähernd NA = 1 beträgt, spielt außerdem eine eventuelle Rauheit der Kante K keine Rolle. Nachteil dieser Ausführungsform ist, dass das aus dem Wellenleiter W austretende Licht in z-Richtung divergiert, was über die (daher geringzuhaltende) Strecke s zwischen Kante K und Detektor DT zu einer Strahlaufweitung und somit zu einer schlechteren Rauschunterdrückung in z-Richtung führt.

Im Ausführungsbeispiel der Figur 7 ist daher eine Zylinderlinse L2 zur Abbildung der Kante auf den Detektor eingefügt, welche das in z-Richtung divergent austretende Licht L auf den Detektor DT fokussiert und so die Lichtintensität auf dem Detektor DT erhöht. Hierbei wird der Abstand s zwischen der Kante K des Wellenleiters W und dem Detektor DT passend zur Brennweite der Zylinderlinse in z-Richtung gewählt (beispielsweise so, dass s der vierfachen Brennweite entspricht), und die Brennweite f des Gitters G wird so gewählt, dass der Fokus in y-Richtung weiterhin auf dem Detektor DT liegt.

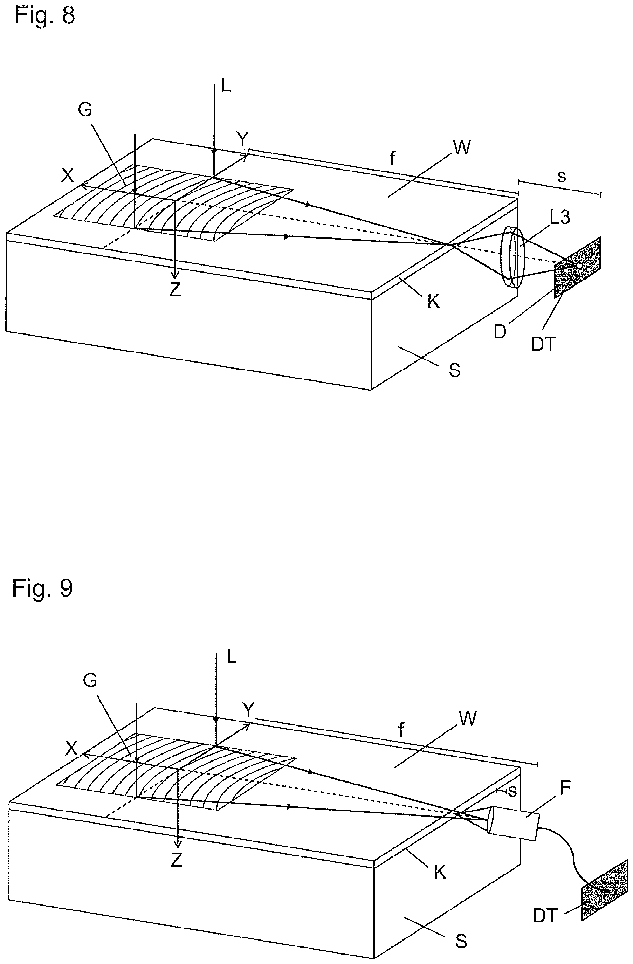

Gemäß der in Figur 8 gezeigten Ausführungsform wird eine sphärische oder asphärische Linse L3 verwendet. Hierbei wird die Brennweite f des Gitters G so gewählt, dass das Signallicht L auf die Kante K des Wellenleiters fokussiert wird. Mit Hilfe der sphärischen oder asphärischen Linse L3 wird die Kante K auf den Detektor DT abgebildet. Der Vorteil dieser Ausführungsform ist die Filterung des Lichts L. Streulicht, in einem Winkel größer als die numerische Apertur der abbildenden sphärischen oder asphärischen Linse L3 kann nicht auf den Detektor DT auftreffen.

Im Zusammenhang mit so einer abbildenden Linse L3 bietet sich insbesondere die Detektion mit einem zweidimensionalen Detektorarray (Kamera) an, siehe weiter unten. Des Weiteren kann durch eine Abbildungsoptik mit einer Blende in einer Fourier-Zwischenebene einfach eine Richtungsfilterung des Signals vorgenommen werden, wie ebenfalls noch näher erläutert wird.

In einer weiteren, in der Figur 9 gezeigten Ausführungsform wird das Signallicht L mit einer lichtleitenden Faser F zum Detektor DT geführt. Hierbei wird die Brennweite f des Gitters G so gewählt, dass das austretende Licht L in y-Richtung auf das Ende einer lichtleitenden Faser fokussiert ist, welches sich in möglichst geringem Abstand s (z.B. zwischen 10 und 100 Mikrometer) zur Kante K des Wellenleiters W befindet. Diese Ausführungsform ist ähnlich zur bereits diskutierten linsenlosen Ausführungsform der Figur 6, die Lichtleitung in der Faser F bietet aber den zusätzlichen Vorteil, dass sich der Detektor DT räumlich getrennt vom Substrat S befinden kann, was eine flexiblere Anordnung der Bestandteile eines Biosensors ermöglicht. Um die Nachteile des an der Kante K in z-Richtung divergent austretenden Lichts zu beheben, kann diese Ausführungsform wiederum mit den bereits diskutierten Zylinderlinsen (Figur 7) oder sphärischen/asphärischen Linsen (Figur 8) kombiniert werden.

Die in den Figuren 6 - 9 gezeigten Ausführungsformen zur Abbildung des an der Kante K austretenden Signallichts L wurden zur besseren Übersichtlichkeit jeweils für nur ein Gitter G diskutiert, lassen sich aber im Fall von Multiplexing mit m x n Gittern Gm.n (Figuren 3, 4a, 4b, 5) problemlos auf entsprechende 1 x n Arrays (Linsenarray, Faserarray, Detektorarray) erweitern. Das bedeutet, dass die in den Figuren 6 - 9 gezeigten Abbildungsmöglichkeiten für jede der n Spalten mit m Gittern Gm.n wiederholt werden. Alternativ kann auch mit nur einer Optik für das gesamte Bildfeld gearbeitet werden. Die verwendeten optischen Elemente (z.B. Linsen, Fasern, Wellenleiter) können sowohl aus geeigneten Gläsern als auch Kunststoffen oder Kombinationen davon bestehen.

Für die Detektoren DT in allen Ausführungsbeispielen gilt: Die Sensorgeometrie kann als einzelnes Pixel, eindimensionales Pixelarray oder zweidimensionales Pixelarray ausgeführt werden.

Die Ausführungsform als einzelnes Pixel, beispielsweise realisiert durch eine Fotodiode, ist besonders vorteilhaft um eine einfache, kostengünstige Detektionseinheit zu erreichen. Auch eine Verwendung im Zusammenhang mit Multiplexing eines m x n - Arrays von Gittern Gm.n, die sequentiell beleuchtet werden und alle auf denselben Fokuspunkt zeigen, ist mit nur einem Detektorpixel möglich.

Eine zweite Ausführungsform ist die Verwendung eines eindimensionalen Zeilenarrays von Detektoren DT, die entlang der auskoppelnden Kante K des Wellenleiters W angeordnet werden, sodass das Licht der n Spalten von Gittern Gm.n auf verschiedene räumlich getrennte Detektorpixel fällt. Es können dabei auch mehr als n Pixel verwendet werden, und die örtliche Filterung kann in einer Auswertesoftware erfolgen.

Eine weitere Möglichkeit besteht in der Verwendung eines zweidimensionalen Detektorarrays. Wie schon oben erwähnt, bietet sich diese Variante besonders in Kombination mit einer abbildenden sphärischen oder asphärischen Linse L3 an, da man so eine Kamera erhält, die die Kante K des Wellenleiters W abbildet. Auf diese Weise kann man den Fokus des austretenden Signallichts L detektieren und erhält gleichzeitig Information über den Streulichthintergrund in y- und z-Richtung, und kann somit das Signal räumlich am Detektor DT filtern.

Wie schon weiter oben angedeutet, ist es vorteilhaft einen eventuell entstehenden Streulichtuntergrund vom zu detektierenden Signallicht zu trennen. Während ein strukturierter Fotodetektor (Pixelarray) bereits intrinsisch zu einer räumlichen Filterung des Detektorsignals führt, kann es von Vorteil sein, das Signallicht L zusätzlich zu filtern um Streulicht zu unterdrücken.

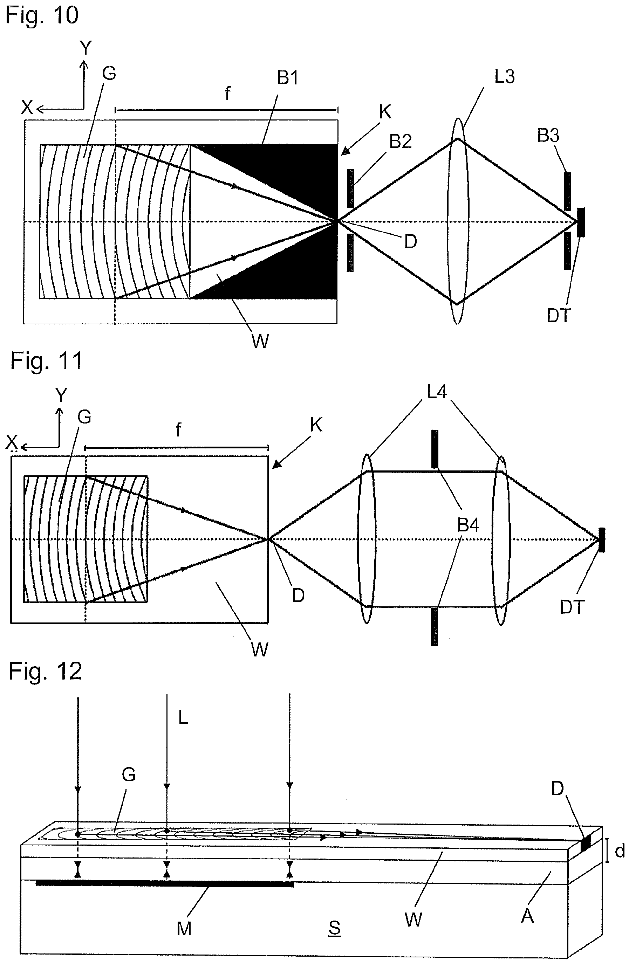

Eine mögliche Ausführungsform ist die in Figur 10 gezeigte Filterung im Ortsraum. Es können ganz einfach eine oder zwei Ortsraumblenden B2, B3 an den in der Figur 10 markierten Positionen angebracht werden, also zwischen der Kante K und der abbildenden Linse L3 und/oder zwischen der Linse L3 und dem Detektor DT. Dadurch wird die Apertur des Detektors DT maskiert, so dass ein kleinerer Bereich der Kante K des Wellenleiters W abgebildet wird.

Auch eine in den Biosensor integrierte Blende B1 ist möglich. Hierzu kann eine zusätzliche, vorzugsweise absorbierende Schicht (z.B. Chrom) auf den Wellenleiter W aufgebracht werden, welche die Totalreflektion im Wellenleiter W verhindert und so das abzublendende Licht auskoppelt und gleichzeitig absorbiert. Hierbei ist zu beachten, dass so eine Blende B1 keine reine Ortsraumblende mehr ist, da sie auch einen Teil des Winkelspektrums beeinflusst.

Figur 11 zeigt eine weitere mögliche Ausführungsform für die Unterdrückung von Streulicht, hier durch eine Filterung im Fourierraum. Durch eine Abbildungsoptik L4 mit mehreren Linsen mit einer Blende B4 in einer Fourier-Zwischenebene kann bequem eine Richtungsfilterung des Signallichtes L vorgenommen werden.

Zu den bisher gezeigten Ausführungsbeispielen sei noch ergänzt, dass die Beleuchtung nicht nur wie gezeigt mediumseitig (von oben) erfolgen kann, sondern auch substratseitig (von unten).

Figur 12 zeigt ein weiteres Ausführungsbeispiel, bei dem die Einkoppeleffizienz des Gitters G deutlich verbessert ist. Hierfür ist eine planare, spiegelnde Schicht (Spiegelschicht) M vorgesehen, die in einem gegebenen Abstand d, definiert durch eine geeignete transparente Abstandsschicht A, auf der dem Lichteinfall abgewandten Seite des Wellenleiters W aufgebracht wird. Hierbei kann es sich sowohl um einen metallischen als auch um einen dielektrischen (z. B. Distributed Bragg Reflector, kurz DBR) Spiegel M handeln. Der Spiegel M hat die Funktion, das im ersten Durchlauf durch das Gitter G hindurch transmittierte Licht zu diesem zurück zu reflektieren, um es ein zweites Mal mit dem Gitter G wechselwirken zu lassen.

Hierzu ist der Abstand d zwischen dem Gitter G und dem Spiegel M so zu wählen, dass konstruktive Interferenz zwischen den beiden Teilstrahlen herbeigeführt wird, was die Einkoppeleffizienz und somit das Signal im Detektor DT um einen Faktor 4 erhöht.

## Patentansprüche

1. Vorrichtung zum selektiven Nachweis von Biomolekülen, umfassend eine Lichtquelle und einen diffraktiven Biosensor mit einem Substrat (S) und einem auf dem Substrat (S) angeordneten flächigen Wellenleiter (W), mit einem auf dem Wellenleiter (W) angeordneten optischen Gitter (G) zum Einkoppeln von einfallendem Licht (L) der Lichtquelle in den Wellenleiter (W), wobei das einfallende Licht (L) kollimiert ist, und einem hinter einer Kante (K) des Wellenleiters (W) liegenden Detektionsbereich (D), wobei der Wellenleiter (W) eingerichtet ist, das Licht (L) zum Detektionsbereich (D) zu leiten, wobei die Effizienz der Einkopplung und damit die Intensität des im Detektionsbereich (D) eintreffenden Lichtes (L) von einer Massenbelegung des optischen Gitters (G) mit den nachzuweisenden Biomolekülen abhängt, **dadurch gekennzeichnet, dass** das optische Gitter (G) auf dem Wellenleiter (W) periodisch angeordnete Rezeptoren (R) für die Biomoleküle aufweist, und dass das optische Gitter (G) gekrümmte Gitterlinien aufweist und eingerichtet ist, das Licht (L) in Richtung des Detektionsbereiches (D) zu fokussieren.

2. Vorrichtung nach Anspruch 1, wobei der Biosensor einen Detektor (DT) und/oder ein optisches Element (L2, L3, L4, F) umfasst, wobei entweder der Detektor (DT) im Detektionsbereich (D) angeordnet ist, oder das optische Element (L2, L3, L4, F) das Licht (L) vom Detektionsbereich (D) auf den Detektor (DT) abbildet.

3. Vorrichtung nach Anspruch 2, wobei das optische Element eine lichtleitende Faser (F) ist.

4. Vorrichtung nach Anspruch 1 oder 2, wobei der Biosensor einen oder mehrere Detektionsbereiche (D) umfasst und auf dem Wellenleiter (W) mehrere optische Gitter (Gm.n) angeordnet sind, die eingerichtet sind, das Licht (L) räumlich und/oder zeitlich getrennt zu einem oder mehreren Detektionsbereichen (D) des Biosensors zu leiten.

5. Vorrichtung nach Anspruch 4, wobei von einem der optischen Gitter (Gm.n) in den Wellenleiter (W) eingekoppeltes Licht (L) Bereiche des Wellenleiters (W) passiert, die weitere optische Gitter (Gm.n) tragen.

6. Vorrichtung nach Anspruch 4 oder 5, wobei die Vorrichtung eine rotierende Blende mit einer oder mehreren Öffnungen (NO) umfasst, die so eingerichtet ist, dass das Licht (L) mit zeitlichem Abstand auf mehrere optische Gitter (Gm.n) fällt.

7. Vorrichtung nach Anspruch 4, 5 oder 6, wobei der Biosensor mehrere getrennt voneinanger liegende Detektionsbereiche (D) umfasst und die mehreren optischen Gitter (Gm.n) das Licht (L) in unterschiedliche Richtungen zu getrennt voneinander liegenden Detektionsbereichen (D) leiten.

8. Vorrichtung nach einem der Ansprüche 2 bis 7, wobei die Vorrichtung eine oder mehrere Blenden (B1, B2, B3, B4) zum Blockieren des einfallenden Streulichts umfasst, die so angeordnet sind, dass das Licht (L) auf dem Weg vom optischen Gitter (G, Gm.n) zum Detektionsbereich (D) oder Detektor (DT) durch die eine oder mehrere Blenden (B1, B2, B3, B4) fällt.

9. Vorrichtung nach einem der vorhergehenden Ansprüche, wobei das Licht (L) von der dem Wellenleiter (W) zugewandten Seite her auf den Biosensor fällt, und dass zwischen dem Wellenleiter (W) und dem Substrat (S) eine Spiegelschicht (M) angeordnet ist, die nicht in den Wellenleiter (W) eingekoppeltes Licht (L) zurück zum optischen Gitter (G) reflektiert.

10. Vorrichtung nach Anspruch 9, wobei zwischen dem Wellenleiter (W) und der Spiegelschicht (M) eine Abstandsschicht (A) angeordnet ist, deren Dicke (d) so gewählt ist, dass erstmals auf das optische Gitter (G) fallendes Licht und von der Spiegelschicht (M) reflektiertes Licht (L) am optischen Gitter (G) konstruktiv interferieren.

## Claims

1. Apparatus for selectively detecting biomolecules, said apparatus comprising a light source and a diffractive biosensor with a substrate (S) and a planar waveguide (W) disposed on the substrate (S), with an optical grating (G), disposed on the waveguide (W), for coupling light (L) incident from the light source into the waveguide (W), the incident light (L) being collimated, and a detection region (D) located behind an edge (K) of the waveguide (W), wherein the waveguide (W) is configured to qiaide the light (L) to the detection region (D), wherein the efficiency of the input coupling and hence the intensity of the light (L) incident in the detection region (D) depends on a surface den of the biomolecules to be detected on the optical grating (G), **characterized in that** the optical grating (G) has receptors (R) for the biomolecules, which receptors are periodically arranged on the waveguide (W), and **in that** the optical grating (G) has curved grating lines and is configured to focus the light (L) in the direction or the detection region (D).

2. Apparatus according to Claim 1, wherein the biosensor comprises a detector (DT) and/or an optical element (L2, L3, L4, F), wherein either the detector (DT) is arranged in the detection region (D) or the optical element (L2, L3, L4, F) images the light (L) from the detection region (D) on the detector (DT).

3. Apparatus according to Claim 2, wherein the optical element is a light-guiding fibre (F).

4. Apparatus according to Claim 1 or 2, wherein the biosensor comprises one or more detection regions (D) and one or more optical gratings (Gm.n) are disposed on the waveguide (W), said optical gratings being configured to guide the light (L) in spatially and/or temporally separated fashion to one or more detection regions (D) of the biosensor.

5. Apparatus according to Claim 4, wherein light (L) coupled into the waveguide (W) by one of the optical gratings (Gm.n) passes through regions of the waveguide (W) that support further optical gratings (Gm.n).

6. Apparatus according to Claim 4 or 5, wherein the apparatus comprises a rotating stop with one or more apertures (NO), which are configured such that the light (L) is incident on a plurality of optical gratings (Gm.n) with a time lag.

7. Apparatus according to Claim 4, 5 or 6, wherein the biosensor comprises a plurality of spaced apart detection regions (D) and the plurality of optical gratings (Gm.n) guide the light (L) in different directions to detection regions (D) that are separated from one another.

8. Apparatus according to any one of Claims 2 to 7, wherein the apparatus comprises one or more stops (B1, B2, B3, B4) for blocking the incident stray light, which are arranged in such a way that, the light (L) travelling from the optical grating (G, Gm.n) to the detection ion (D) or detector (DT) passes through the one or more stops (B1, B2, B3, B4).

9. Apparatus according to any one of the preceding claims, wherein the light (L) is incident on the biosensor from the side facing the waveguide (W) and in that a mirror layer (M) is disposed between the waveguide (W) and the substrate (S), said mirror layer reflecting light (L) not coupled into the waveguide (W)back to the optical grating (G).

10. Apparatus according to Claim 9, wherein a spacer layer (A) is disposed between the waveguide (W) and the mirror layer (M), the thickness (d) of said spacer layer being chosen such that light incident on the optical grating (G) for the first time and light (L) reflected by the mirror layer (M) interfere constructively at the optical grating (G).

**Revendications**

1. Dispositif de détection sélective de biomolécules, comprenant une source lumineuse et un biocapteur diffractif pourvu d'un substrat (S) et d'un guide d'ondes (W) sensiblement bidimensionnel disposé sur le substrat (S), d'un réseau optique (G) disposé sur le guide d'ondes (W) et destiné à injecter de la lumière incidente (L) de la source lumineuse dans le guide d'ondes (W), la lumière incidente (L) étant collimatée, et d'une zone de détection (D) située derrière un bord (K) du guide d'ondes (W), le guide d'ondes (W) étant conçu pour guider la lumière (L) vers la zone de détection (D), le rendement de l'injection et donc l'intensité de la lumière (L) arrivant dans la zone de détection (D) dépendant d'une occupation massique du réseau optique (G) par les biomolécules à détecter, **caractérisé en ce que** le réseau optique (G) comporte des récepteurs (R) disposés périodiquement sur le guide d'ondes (W) et destinés aux biomolécules, et **en ce que** le réseau optique (G) comporte des lignes de réseau incurvées et est conçu pour focaliser la lumière (L) en direction de la zone de détection (D).

2. Dispositif selon la revendication 1, le biocapteur comprenant un détecteur (DT) et/ou un élément optique (L2, L3, L4, F), le détecteur (DT) étant disposé dans la zone de détection (D) ou bien l'élément optique (L2, L3, L4, F) reproduisant la lumière (L) de la zone de détection (D) sur le détecteur (DT).

3. Dispositif selon la revendication 2, l'élément que étant une fibre optique (F).

4. Dispositif selon la revendication 1 ou 2, le biocapteur comprenant une ou plusieurs zones de détection (D) et plusieurs réseaux optiques (Gm.n) étant disposés sur le guide d'onde (W), lesquels sont adapter pour guider la lumière (L) de manière séparée spatialement et/ou temporeilement vers une ou plusieurs zones de détection (D) du biocapteur.

5. Dispositif selon la revendication 4, la lumière (L) injectée par l'un des réseaux optiques (Gm.n) dans le guide d'ondes (W) passant par des régions du guide d'ondes (W) qui portent d'autres réseaux optiques (Gm.n).

6. Dispositif selon la revendication 4 ou 5, le dispositif comprenant. un diaphragme rotatif pourvu d'une ou plusieurs ouvertures (NO) et conçu de telle sorte que la lumière (L) est incidente à plusieurs réseaux optiques (Gm.n) suivant un intervalle de temps.

7. Dispositif selon la revendication 4, 5 ou 6, le biocapteur comprenant une pluralité de zones de détection (D) situées séparément les unes des autres et la pluralité de réseaux optiques (Gm.n) guidant la lumière (L) dans différentes directions vers des zones de détection (D) situées séparément les unes des autres.

8. Dispositif selon l'une des revendications 2 à 7, le dispositif comprenant un ou plusieurs diaphragmes (B2, B3, B4) destinés à bloquer la lumière diffusée incidente et disposés de telle sorte que la lumière (L) est incidente au chemin allant du réseau optique (G, Gm.n) à la zone de détection (D) ou au détecteur (DT) par le biais des un ou plusieurs diaphragmes (B1, B2, B3, B4) .

9. Dispositif selon l'une des revendications précédentes, la lumière (L) étant incidente au biocapteur depuis le côte dirige vers le guide d'ondes (W), et en ce qu'une couche miroir (M) est disposée entre le guide d'ondes (W) et le substrat (S) et réfléchit vers le réseau optique (G) la lumière (L) qui n'a pas été injectée dans le guide d'ondes (W).

10. Dispositif selon la revendication 9, une couche d'espacement (A) étant disposée entre le guide d'ondes (W) et la couche miroir (M), l'épaisseur (d) de la couche d'espacement étant choisie de manière à ce que la lumière incidente pour la première fois au réseau optique (G) et la lumière (L) réfléchie par la couche miroir (M) interfèrent de manière constructive au niveau du réseau optique (G).

## Fig. 1

## Fig. 2

Fig. 3

Fig. 4a

## Fig. 4b

## Fig. 5

Fig. 6

Fig. 7

Fig. 8

Fig. 9

Fig. 10

Fig. 11

Fig. 12

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20140080729 A1
- WO 2010063116 A1
- US 7505641 B1
- WO 9737211 A1
- WO 2015004264 A1
- WO 2013107811 A1
- EP 0226604 B1